(19) Europäisches Patentamt
European Patent Office
Office européen des brevets

(11) Numéro de publication: **0 079 297 A1**

(12) # DEMANDE DE BREVET EUROPEEN

(21) Numéro de dépôt: 82810343.2

(22) Date de dépôt: 13.08.82

(51) Int. Cl.³: C 07 D 233/56
C 07 D 307/52, A 61 K 31/415
A 61 K 31/34

(30) Priorité: 14.08.81 CH 5230/81

(43) Date de publication de la demande: 18.05.83 Bulletin 83/20

(84) Etats contractants désignés: AT BE DE FR GB IT NL SE

(71) Demandeur: Société de Recherches et de Synthèses Organiques SA
12 rue Marziano
CH-1227 Genève(CH)

(72) Inventeur: Baudet, Pierre Jean
15 chemin de Passoret
CH-1227 Genève(CH)

(54) **Sels de l'acide amido-sulfonique.**

(57) Monosels di-sels et sels mixtes de l'acide sulfamique caractérisé par un composé de formule (I)

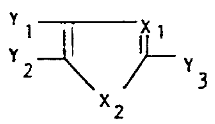

dans laquelle:
- quand $X_1$ et $X_2$ = N alors

$Y_1 = -CH_2SCH_2CH_2N{=}C\begin{smallmatrix}NHCN\\NHCH_3\end{smallmatrix}$

$Y_2 = -CH_3$

$Y_3 = -H$

- quand $X_1 = -CH-$

$X_2 = -O-$ alors

$Y_1 = -H$

$Y_2 = -CH_2\ N(CH_3)_2$

$Y_3 = -CH_2SCH_2CH_2N{=}C\begin{smallmatrix}NHCN\\NHCH_3\end{smallmatrix}$

leurs méthodes de préparation et leurs applications en gastro-entérologie, dans la thérapie des ulceres du duodenum et de l'estomac.

EP 0 079 297 A1

Croydon Printing Company Ltd

DESCRIPTION

## Sels de l'acide amido-sulfonique

Les alkyl-urées, les alkyl-thiourées, les alkyl-carbamates, les guanidines alkylées, les alkyl-amidines, certaines amines secondaires sont N-nitrosées dans des milieux biologiques de pH 1 à pH 4, fournissant des composés N-nitroso, dont la décomposition en intermédiaire à carbocation se sont révélés cancérigènes. L'estomac est un organe dans lequel la N-nitrosation des composés indiqués plus haut peut s'effectuer parce que:
(1) l'alimentation contient des nitrites alcalins, agents de la nitrosation et des nitrates alcalins susceptibles de réduction bactériologique en nitrites alcalins,
(2) le pH acide du milieu gastrique est favorable à la nitrosation des ccomposés N-nitrosables,
(3) le bol alimentaire contient des composés nitrosables (aliments et médicaments).
Un traitement médicamenteux comportant un ou plusieurs composés N-nitrosables est donc susceptible de produire, dans le milieu gastrique, des composés potentiellement cancérigènes.
Il est donc necessaire d'associer à l'agent thérapeutique N-nitrosable, un composé qui empêche la réaction de nitrosation dans le milieu gastrique. Il paraît également néccessaire de découvrir un composé empêchant la nitrosation, dont la réaction spécifique ne fournit pas de mono-oxyde d'azote.
La cimétidine est imino-cyano-guanidine appartenant à la catégorie des médicaments N-nitrosables. A pH 1, la vitesse initiale de sa nitrosation est de 3.970 $mol.l^{-1}$ $S^{-1}$. Une solution de chlorhydrate de cimétidine dans de l'eau, de pH 1,0, 0 1 $mol.l^{-1}$ contenant 0,04 $mol.l^{-1}$ de nitrite de sodium, à 37°, fournit en une heure 42,5 % de N-nitroso-méthyl-N'-imino-cyano-N''-[2((4-méthyl-5-imidazolyl)-méthylthio)-éthyl]-guanidine. Aux pH 1,92 3,04 et 4,01 le % de ce composé est respectivement de 15,3 1,7 et 0,15 % (J.Chem.Res, (5).212-213 (1980)).
La nitrosation de la mono-méthyl urée (5 mmoles), en présence de nitrite de Na (10mmoles), à pH 1, à 25°, en 10 min. fournit 85 % de N-nitroso-N-méthyl urée; à pH 2 toutes les autres conditions étant les mêmes, 58 % de la nitroso-urée est obtenu.
L'équation de réaction de l'acide nitreux avec les composés

BAD ORIGINAL

N-nitrosables,entre pH 1 et 2 est :

$V=k.[NO_2H].[substrat].[H^+]$

Aux pH supérieurs à 2,un autre mécanisme se manifeste,dont la vitesse initiale est notablement réduite par rapport à la vitesse de réaction du mécanisme prévalant aux pH plus bas.

Le facteur limitant la N-nitrosation,dans le milieu gastrique, est la concentration des nitrites alcalins.Cette concentration dépend:(a) de la nature du bol alimentaire,

(b) de la possibilité de réduction,in situ,des nitrates alcalins en nitrites alcalins,par action bactérienne.En effet, si le milieu gastrique n'est pas stérile,à la suite de la neutralisation ou de la diminution de la sécrétion (pH 3-4),les bactéries réductrices des nitrates,telles que micrococcus spp. staphylococcus aureus etc,peuvent s'y développer.

La concentration des nitrites alcalins,dans le milieu gastrique,à jeun,aprés le repas,selon le pH gastrique,est donnée dans le tableau suivant(réf. Lancet 408 21.2.1981)),

| Conc.nitrites | à jeun | | aprés le repas | |
|---|---|---|---|---|
| μ mol./l | pH<4 | pH≥4 | pH<4 | pH≥4 |
| | % | % | % | % |
| 10 | 90 | 10 | 77 | 23 |
| 10-20 | 80 | 20 | 87 | 13 |
| 20 | 22 | 78 | 33 | 67 |

%= des cas examinés.

La nitrosation aux pH 4 devient trés lente.En effet,cette réation s'arrête lorsque la valeur du pH interdit l'hydrolyse des nitrites alcalins en acide nitreux.Il s'en suit que la N-nitrosation des alkylurées,des alkyl.thiourées,des alkylcarbamates.des alkylguanidines et des alkylamidines s'effectue à des vitesses décroissantes de pH 1 à pH 5.

Les composés de l'invention empêchent dans ce domaine de pH et au delà,la nitrosation de ces catégories de composés,en détruisant l'acide nitreux,agent de la nitrosation,sans produire d'oxyde d'azote (NO):

$$NO_2H + NH_2SO_3^- \longrightarrow SO_4H^- + H_2O + N_2$$

Ainsi,les sulfamates(ou amido-sulffonates) de l'invention sont caractérisés en plus de l'activité thérapeutique spécifique

BAD ORIGINAL

de leur base,par une activité thérapeutiquement bénéfique,supplémemtaire,par leur partie acide,empêchant la nitrosation dans le milieu gastrique,de la fonction guanidine ou de leur fonction amidine et éventuellement,empêchant la nitrosation d'autres composés du bol alimentaire.

Les caractéristiques thérapeutiques des sels de l'invention ne permettent pas de les considérer comme de simples sels thérapeutiquement convenables au sens usuel,entendu dans les demandes de brevet conernant les composés à action thérapeutique et salifiables.

Tous les composés de l'invention antagonisent l'activité de l'histamine dans les récepteurs $H_2$ et par lesquels l'histamine stimule l'effet chronotrope sur l'oreilette droite du cobaye) ou l'effet sécrétoire gastrique.

Il est observé en outre,que les composés de l'invention,notamment le disulfamate de la formule II posséde la propriété surprenante pour l'homme de métier d'une vitesse de résoption intestinale,notablement plus grande que celle d'autres sels thérapeutiquement convenables de la base de ce composé.

Les excipients convenables pour la mise en oeuvre pharmaceutique des sels de l'invention sont par ex. le lactose,le saccharose,le talc,la gélatine,la gomme arabique,l'huile d'olive.

Exemple 1

Mono-sulfamate de N-méthyl-N'-cyano-N''-imino-[2-((4-méthyl-5-imidazolyl)-méthylthio)éthyl]-guanidine.

A une suspension de 17,85 g du dichlorhydrate de N-méthyl-N'-cyano-N''-imino[2-((4-méthyl-5-imidazolyl)-méthylthio)-éthyl]-guanidine dans de l'éthanol,on ajoute 6,80 g d'éthoxyde de sodium,dans le même solvant.Aprés compléte transformation du sel dans la base correspondante,on éloigne NaCl et neutralise le filtrat par 4,85 g d'acide sulfamique en solution dans du méthanol.Le mono-sulfamate cristallise.
F.131-133°; titration de $NH_2SO_3H$: 99,77 %.
spectre IR: en page 4

BAD ORIGINAL

Spectre IR(nujol) du mono-sulfamate

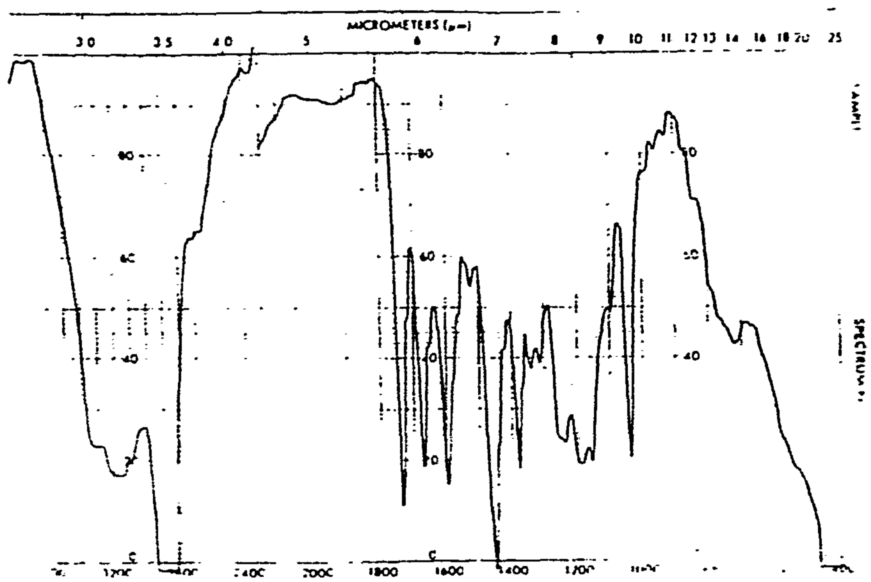

Exemple 2

Di-sulfamate de N-méthyl-N'-cyano-N''-imino[2-((4-méthyl-5-imidazolyl)-méthylthio)-éthyl]-guanidine.

La quantité de base N-méthyl-N'-cyano-N''-imino -2-((4-méthyl-5-imidazolyl)-méthylthio)-éthyl guanidne provenant de la neutralisation de 19,63 g du dichlorhydrate,par 7,48 g d'éthoxyde de sodium,est neutralisée dans de l'éthanol,par 10,67 g d'acide sulfamique,dissous dans du méthanol.Le di-sulfamate cristallise.

F.141-142°; titration de $NH_2SO_3H$: 99,91 %.

Spectre IR(nujol) du di-sulfamate

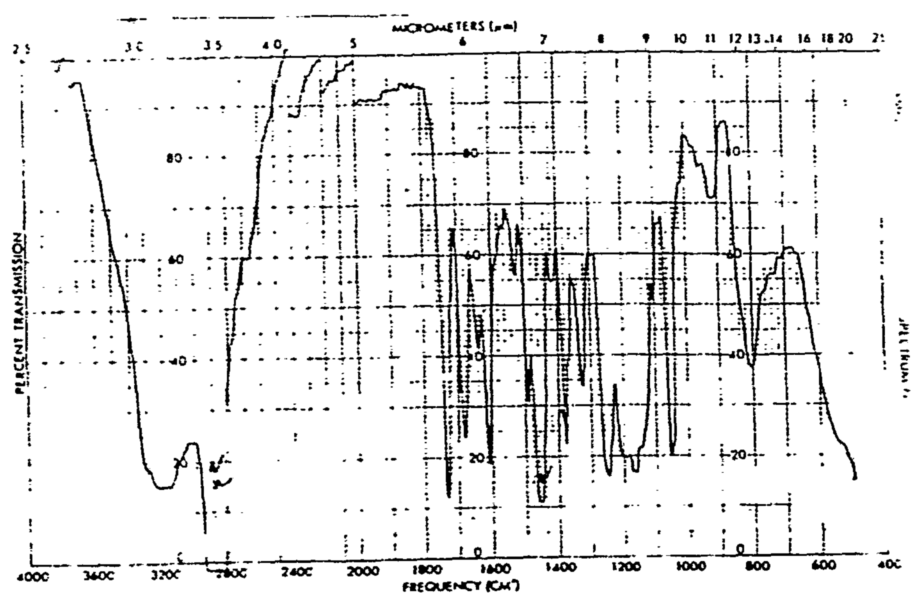

Exemple 3

Mono-sulfamate de N-méthyl-N'-cyano-N''-imino [2-[[5-(diméthylamino)-méthyl-2-furanyl]-méthylthio] -éthyl]-guanidine.

La quantité de base de N-méthyl-N'-cyano-N''-imino 2- 5-(diméthylamino)-méthyl-2-furanyl -méthylthio -éthyl - guanidine en solution dans de l'éthanol,provenant de la neutralisation de 6,32 g du dichlorhydrate,par 2,40 g d'éthoxyde de sodium,est neutralisé par 1,72 g d'acide sulfamique dissous dans du méthanol.L'évaporation du solvant fournit un solide.Titration de $NH_2SO_3H$:99,7 %; Spectre IR(nujol):caractéristique de la fonction N-méthyl-N'-cyano-N''-imonium-R guanidine,R=2- 5-(diméthylamino)-méthyl-2-furanyl -méthylthio -éthyle)

Exemple 4

Di-sulfamate de N-méthyl-N'-cyano-N''-imino[2-[[5-(diméthylamino)-méthyl-2-furanyl]-méthylthio]-éthyl]-guanidine.

La quantité de base provenant de la neutralisation de 3,68 g de dichlorhydrate de N-méthyl-N'-cyano-N''-imino- 2- 5-(diméthylamino)-méthyl-2-furanyl -méthylthio - éthyl -guanidine,par 1,36 g d'éthoxyde de sodium,est neutralisée dans de l'éthanol par 1,94 g d'aide sulfamique dissous dans du méthanol.L'évaporation du solvant fournit un solide;titration de $NH_2SO_3H$:99,85 %;spectre IR(nujol): caractéristique de la fonction N-méthyl-N'-cyano-N''-iminium-R guanidine,R= 2- 5-(diméthylamino)-méthyl-2-furanyl - méthylthio -éthyle.

REVENDICATIONS

1. sel de l'acide sulfamique caractérisé par le composé de la formule:

I 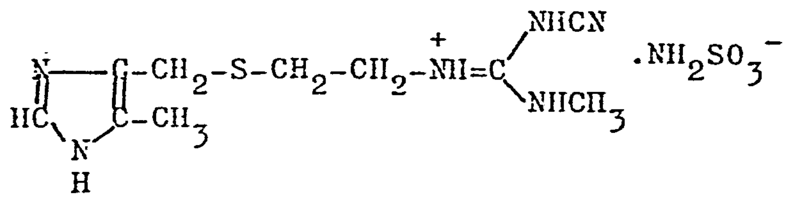

structure imino-alkyle et amino-nitrile,

2. selde l'acide sulfamique caractérisé par le composé de la formule:

II 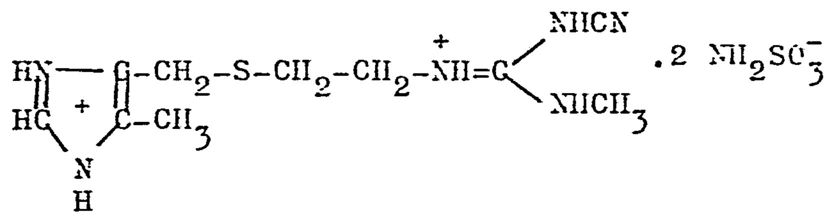

3. sels mixtes de l'acide sulfamique et d'un acide minéral mono-acide différent caractérisés par les composés de la formule:

III 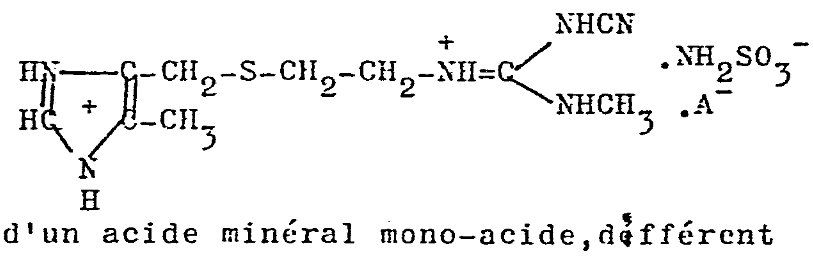

$A^-$ = reste anion d'un acide minéral mono-acide, différent de l'acide sulfamique,

4. sels mixtes de l'acide sulfamique et d'un acide mono-carboxylique ou d'un acide mono-sulfonique organique ou d'un acide ester-phosphorique organique, caractérisés par les composés de la formule:

IV 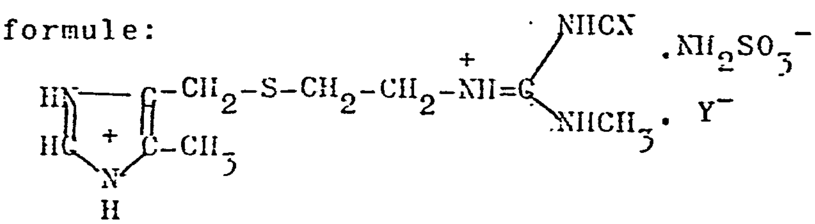

$Y^-$ = reste anion d'un acide mono-carboxylique ou d'un reste anion acide mono-sulfonique organique ou d'un reste mono-phosphorique organique,

5. sel de l'acide sulfamique,caractérisé par le composé de la formule:

V 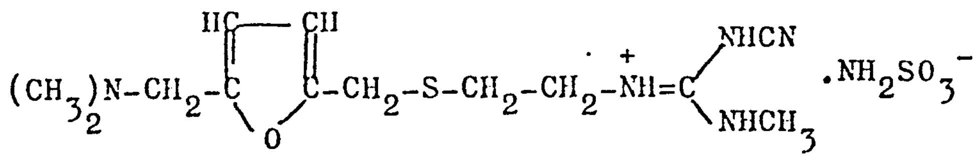

6. sel de l'acide sulfamique,caractérisé par le composé de la formule:

VI 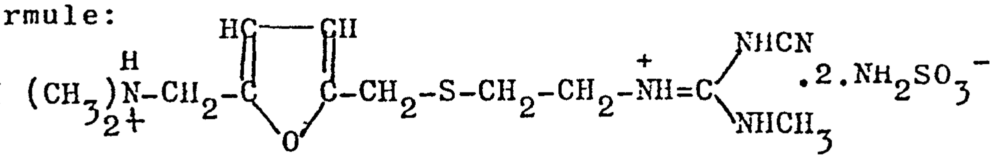

7. sels mixtes de l'acide sulfamique et d'un autre mono-acide minéral,caractérisé par les composés de la formule:

VII 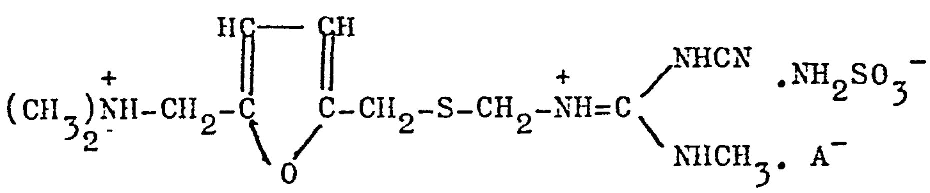

$A^-$ = le reste anion d'un mono-acide minéral,différent de l'acide sulfamique,

8. Procédé selon les revendications 1,2,5 et 6 pour la préparation des composés des formules I,II,V et VI, caractérisé par ce que l'on neutralise les bases des composés des formules des formules I et V,par un équivalent d'acide sulfamique,dans un solvant convenable,tel par ex. l'éthanol,à partir duquel les sulfamates cristallisent, par ce que l'on neutralise les bases des composés des formules II et VI par deux équivalents d'acide sulfamique,dans un solvant convenable,par ex. l'éthanol.à partir duquel les di-sulfamates cristallisent.

9. Procédé selon les revendications 1,2,5 et 6 pour la préparation des composés des I,II,V et VI, caractérisé par ce que l'on effectue une double décomposition entre un sel monocarboxylique,par ex. un acétate, des bases des composés des formules I et V et un équivalent d'un sulfamate alcalin,dans un solvant convenable,par ex.

l'éthanol,à partir duquel les sulfamates cristallisent, caractérisé par ce que l'on effectue une double décomposition entre un di-sel mono-carboxylique,par ex.un acétate, des bases des composés des formules II et VI et deux équivalents d'un sulfamate alcalin,dans un solvant convenable, par ex. l'éthanol,à partir duquel les di-sulfamates cristallisent.

10. Procédé selon les revendications 3 et 7,pour la préparation des composés des formules III et VII,
caractérisé par ce que l'on neutralise le composé de la formule I par un équivalent d'un mono-acide minéral,
ou,
caractérisé par ce que l'on neutralise le sel d'un mono-acide minéral de la base du composé de la formule I,par un équivalent d'acide sulfamique,
caractérisé par ce que l'on neutralise le composé de la formule V par un équivalent d'un mono-acide méneral,
ou,
caractésisé par ce que l'on neutralise le sel d'un mono-acide minéral de la base du composé de la formule V,par un équivalent d'acide sulfamique.

Office europeen des brevets

**RAPPORT PARTIEL DE RECHERCHE EUROPEENNE**

qui selon la regle 45 de la Convention sur le brevet europeen est considere, aux fins de la procédure ulterieure, comme le rapport de recherche europeenne

0079297

Numéro de la demande

EP 82 81 0343

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int. Cl. 3) |
|---|---|---|---|
| A | EP - A - 0 001 699 (GLAXO) | | C 07 D 233/56<br>307/52<br>A 61 K 31/415<br>31/34 |
| A | WO - A - 81/00255 (AU NOM DU DEPOSANTE)<br>--------- | | |

DOMAINES TECHNIQUES RECHERCHES (Int. Cl. 3)

C 07 D 233/00
307/00
A 61 K 31/00

## RECHERCHE INCOMPLETE

La division de la recherche estime que la presente demande de brevet europeen n'est pas conforme aux dispositions de la Convention sur le brevet europeen au point qu'une recherche significative sur l'etat de la technique ne peut être effectuee au regard d'une partie des revendications.

Revendications ayant fait l'objet de recherches complètes: 1,2,5,6,8,9

Revendications ayant fait l'objet de recherches incomplètes:

Revendications n'ayant pas fait l'objet de recherches:

Raison pour la limitation de la recherche: 3-4, 7, 10, article 84.

| Lieu de la recherche | Date d'achèvement de la recherche | Examinateur |
|---|---|---|
| La Haye | 16 novembre 1982 | DE BUYSER |

CATEGORIE DES DOCUMENTS CITES

- X : particulièrement pertinent à lui seul
- Y : particulièrement pertinent en combinaison avec un autre document de la même categorie
- A : arrière-plan technologique
- O : divulgation non-ecrite
- P : document intercalaire
- T : theorie ou principe à la base de l'invention
- E : document de brevet antérieur, mais publie à la date de depôt ou apres cette date
- D : cite dans la demande
- L : cite pour d'autres raisons
- & : membre de la même famille, document correspondant

OEB Form 1505.1 03.82